# EUROPEAN PATENT APPLICATION

(11) **EP 1 062 914 A1**
(43) Date of publication of application: **27.12.2000**
(21) Application number: 99939155.0
(22) Date of filing: 17.02.1999
(51) Int. Cl.: A61B 6/03, G01N 23/02, G21K 1/02

(54) **ULTRA-SMALL-ANGLE X-RAY TOMOGRAPHY**

(30) Priority: 12.03.1998 RU 98104687
(71) Applicant: Quanta Vision, Inc., San Mateo, CA 94402 (US)
(72) Inventor: LAZAREV, Pavel Ivanovich, Moscow, 119633 (RU); Komardin, Oleg, Valentinovich, Moscow, 121467 (RU)
(74) Representative: Godwin, Edgar James
(86) International application number: RU9900042
(87) International publication number: WO9945843

(57) **Abstract**

The invention deals with computerized tomography based on the object imaging with small angle scattered radiation. The registration of the scattering is made in ultra-small angles: from 0 to 1 degree relatively to the direction of the falling beam. Several schemes allowing to perform the registration of coherent scattering in the mentioned angles are suggested. The fan-shaped low-diverging beams formed by the collimator are directed to the object. In one of the variants of the device it is suggested to use a special spatial filter situated beyond the object for the separation of the radiation scattered on small angles; this filter represents a collimator-like structure, in which radiation-transparent areas of the collimator are overlapped by opaque areas of the filter. With this in the absence of the object the radiation does not pass on the space-sensitive detector situated beyond the filter. Being installed between the collimator and the spatial filter, the detector registers the scattering of the radiation on small angles. In order to register the radiation passed through the object in the direction of the primary beam, the detective elements are installed on opaque areas of the filter. It allows to obtain, besides a tomogram in the scattered radiation, tomographic images by the absorbed radiation. In another variant of the device the areas of the filter overlapping the transparent areas of the collimator, are made partially absorbing the radiation passed through the object along the direction of the falling beams, and decreasing it to the level of intensity of the scattered radiation. One of the suggested schemes allows to determine the object's scattering function within the limits of the primary beam. Based on the data of scattering obtained for different views of the object relatively to the system "source - collimator - detector" it is possible to restore the tomographic image of the object. 3 independent points of formulae, 9 figs.

## Description

The invention concerns the devices for computerized tomography based on the absorption and ultra-small angle scattering of x-ray radiation.

The method of computerized tomography (CT) was elaborated in 1973 by the British firm "EMJ" (Hounsfeld G.N., Computerized transverse axial scanning (tomography). Part 1. Description of system. - "Brit.J.Radiol.", 1973, v.46, p.1016-1033; Ambrose J. Computerized transverse axial scanning (tomography). Part 2. Clinical application. - "Brit.J.Radiol.", 1973, v.46, p.1023.-1047). It combines physical principles of traditional x-ray transillumination with the recent achievements of mathematics and digital technique. The essence of the method of computerized tomography consists in the reconstruction of the internal spatial structure of the object as a result of joint mathematical processing of shade projections obtained during x-ray transillumination of the object in different directions. The contrast of each shade projection is the result of unequal x-ray absorption by different parts of the object. The principle of action of the tomograph laid on the method of computerized tomography consists in the following. Right-angled x-ray beam formed by a collimator passes through fixed object on 2 detectors (sodium iodide crystals). The detector registers the amount of radiation passed through the object, and the system x-ray tube - detectors is displaced by one step parallel to itself. A total of 160 displacements is made. Then the system returns to the initial position, turns at 1° angle, and a new scanning of 160 segments is made. A total of 180 turns is performed. The time of the whole turn of the system, that is, the time for receiving the total information is about 5 minutes. With this 288,000 indices are obtained from each detector (160 x 180). The obtained information is processed with a computer. The reconstructed computerized image of a layer is transferred on the calculating-printing device, which gives digital records of absorption coefficients for the whole field of the body section.

Further modernization of CT followed the way of increasing the number of detectors. Computerized tomographs of the III and IV generations contains from 512 to 4800 detectors. With 512 - 1400 detectors and a big capacity computer the time of scanning for one section (2 - 8 mm) decreased to 2 - 5 sec., which practically allowed to examine all the organs and tissues of the organism.

Scanning system of the modern computerized tomograph includes an x-ray tube and a detector system. In the III generation apparatus the x-ray tube and the detectors are situated on the same frame. The detector system consists of 256 - 512 semiconductor elements or xenon detectors. During the scanning of a patient the system "x-ray tube - detectors" rotates around the patient making 360° turn during one cycle. During the rotation of the complex x-ray tube delivers impulse radiation in the form of a fan-shaped beam passing through the object after 1°, 0,5° and 0,25°; with this the detector system registers the radiation weakened by the object. In cases of necessity the scanning system can be inclined forward and backward at 20° - 25°.

In IV generation computerized tomographs the detector system consists of 1400 - 4800 detectors situated circumferentially at a frame. During the scanning process only x-ray tube rotates around the patients. The tomograph table consists of a base and a mobile part with a fixed transporting bed for the patient. Horizontal shift of the patient during the scanning process is performed automatically.

The x-ray system of the tomograph consists of a tube and a generator. The tube works in the impulse regimen at 100-130 kV tension. The absorption of the soft component of the roentgen radiation is realized by filtration, there is a diaphragm at the tube output, which limits the flow of penetrating radiation falling on the object.

As it has been mentioned, the principle of different absorption of x-ray radiation by different materials lays at the base of all the devices described above. For this reason during the investigation of a body consisting of substances differing by their composition and/or structure, but having close or equal coefficient of x-rays absorption, a device based on such principle would not distinguish such substances, that is, the restored image would not contain the information concerning them. For this reason in such cases it is necessary to use another approach to the imaging, based on the principles, which differ from x-rays absorption, on the other type of interaction between the roentgen radiation and the substance.

The patent EP 0784202, 1997 describes a computerized tomograph based on roentgen phase-contrast method, which uses the effect of x-ray refraction at the frontier of the object's areas with different electron density. It leads to x-ray deviation at the angles measuring several seconds. In the suggested device the radiation flow falling on the object is formed with the help of a monocrystal as a parallel beam with small angle scattering. During the passage of such beam through the object containing the substances with different electron density, at the frontier of their separation the radiation flow is deviated as a result of refraction at the angle mentioned above. This deviation is fixed during the turn of a crystal-detector installed beyond the object with the help of a detector.

Among the disadvantages of the phase-contrast method one can mention the fact, that it does not characterize the substance itself but rather the frontier of separation of two substances having different coefficients of x-ray refraction. The formation of the penetrating radiation flow is realized following a bicrystal scheme, and it imposes some limitations on the effectiveness of the use of the radiation source energy. It is caused by the fact that the monocrystal reflects the falling radiation in conformity with Bregg's law. The radiation of each wave's length is reflected at a determined angle within the interval of divergence equal to the Bregg angled interval of reflection, which is of about 10 angled seconds. That means, that less than 10⁸ of the energy from all the radiation produced by the source is used for the object transillumination. It leads to the increase of the exposition time. The use of bicrystal scheme imposes the limitations on the size of the investigated object's area, which is determined by the crystals' size, or a complex scanning system is needed for the imaging of the whole object.

The mentioned disadvantages can be avoided with the use of the method of registration of radiation coherently scattered by the object, for the restoration of tomographic image. The patent US 4752722, GO1N, 23/22, 1988 describes the device based on the principle of registration of the angled distribution of coherently scattered radiation laid within the angles of 1° - 12° as related to the direction of the falling beam. As it is pointed out in this patent, the biggest part of the elastic scattered radiation is concentrated within the angles of less than 12°, and the scattered radiation has a characteristic angled dependency with well marked maxims, whose position is determined by the irradiated substance itself; as well as by the energy of the falling radiation. As the distribution of the intensity of the coherently scattered radiation in small angles depends on molecular structure of the substance, different substances having equal absorbing capacity (which cannot be differentiated with conventional transillumination), can be distinguished according the distribution of the intensity of the angled scattering of coherent radiation typical for each substance. It is suggested to use narrow collimated beam of mono- or polychromatic radiation for the object irradiation. The measurement of the intensity of coherently scattered radiation is performed with the help of detector system with the resolution by the energy, as well as by the coordinate (the angle of scattering). The detector registers the intensity of coherently scattered rays, coming out of the frontiers of the primary beam within the zone of scattering. In order to obtain the image it is suggested to use the known principles of computerized tomography. With this the absorbed radiation is registered simultaneously with the scattered radiation, thus allowing for the account of the optical thickness at the way of the penetrating beam for each area of the examined object, that is, for receiving the formed curve of coherently scattered radiation.

The described device has a relatively low sensitivity to the radiation scattered in close proximity to the primary beam, as the intensity of the primary beam radiation exceeds significantly the intensity of the scattered radiation and inhibits its registration. Besides, the radiation intensity falls abruptly with the increase of the angle of scattering, so the intensity of coherently scattered radiation with angled diapason of 1° - 12° is rather small, and so it is necessary to have sufficiently high doses of radiation and long exposition time for object examination.

The purpose of the described invention consists on the elaboration of the devices which, firstly, would be more sensitive to the registration of coherently scattered radiation with ultra-small angles (from dozens of second to one degree), and, secondly, necessitate smaller doses of radiation for the examination of the object.

It is well known, that the essential part of coherently scattered radiation is concentrated in the area of central diffraction peak, which lays in the angles of scattering going from 0 to 1 degree relatively to the direction of the primary beam's fall. In this angled diapason is concentrated a characteristic radiation, coherently scattered by the heterogeneity of the electronic structure of the object measuring from several hundreds to several tens of thousands of angstroms, which is normal for the structure of many organic and biological objects/ For this reason we suggest to measure the distribution of coherently scattered radiation namely in this angled interval. The angled diapason, in which coherently scattered radiation is measured depends on the length of the used radiation's wave and structural properties of the material; it can be situated within the limits from several angled seconds to 1 degree relatively to the falling radiation beam. The invention suggests to use a dark field scheme for the measurement of coherently scattered radiation with ultra small angles (from 0 to 1 degree, that is, when in the absence of the studied object the detector registers only background signal, and in its presence - the scattered radiation. Such scheme is more sensitive to the scattered radiation as compared with the light field system, described in the patent 4752722, GO1N 23/22, 1988. As small angled scattering of the x- rays reflects the internal substance structure (the distribution of electron density), the registration of the curve of small angled scattering of the x-rays by the studied object, that is, the dependence of the intensity of the scattered rays on the angle of scattering, allows to restore the picture of electron density distribution within the object under investigation. If the object is not homogenous (that is, consists of different substances), the intensity of the scattered radiation under each angle is composed of the intensities of the rays, scattered by different substances along the way of distribution of the penetrating radiation's beam. During the transillumination of the studied object from different directions the picture of radiation scattering for each direction is registered, and them using the methods of computerized tomography it is possible to restore the curve of small angle scattering (distribution of electron density) for each separate area of the object and, as a final result, the picture of electron density distribution within the whole object, that is, three-dimension image of this object's internal structure.

The principle of receiving the image of the object's internal structure, described above, can be realized in different variants of the device. The main principle for the creation of such devices consists in simultaneous registration of the radiation absorbed and scattered by the object under ultra small angles (from several angled seconds to 1 degree).

The device allowing for the solution of the problem posed in the invention, is a small-angled x-ray tomograph, which includes the source of x-rat radiation, a collimator forming the penetrating radiation beam in the form of a narrow fan-shaped beam (or several beams) with small angled dispersion, a space filter situated beyond the object, and a detector registering the radiation passed through the investigated object. The source of radiation, the collimator, the space filter and the detector can move around the investigated object in order to examine it from different directions.

The system "collimator - space filter - detector" has to be organized such a way, that it could register simultaneously the scattered radiation and the radiation passed through the object without being scattered, for each examined part of the object.

The collimator must form the beams of penetrating radiation with such a width and angled dispersion in one direction, that it could register the scattered radiation within the small angled diapason, that is, that any primary beam's ray scattered by the object under the small angle α went over the frontiers of the primary beam within the registration zone (α can be of several angled seconds), whole in the other direction the beam formed by the collimator must overlap all the investigated area of the object.

From the constructive point of view the collimator can be made in the form of a set of slit diaphragms situated one after another; in the form of two radiation-opaque plates with a clearance between them; according to Kratky scheme, etc. (Bekrenev A.I., Terminasov Yu.S. Apparatus and methods for x-ray analysis, 1980, iss.24, p.100-108; Schelten, W.Hendriks, Appl.Cryst., 1978, 11,p.297-324). It is possible to use slitless collimator for the formation of the beams of micron and submicron width with angled dispersion of several angled minutes. The principle of such collimator's work is based on the effect of x-rat passage by the frontier of separation of two flat polished plates' surfaces with multiple total external reflection (Leykin V.N., Mingazin T.A., Devices and technique of the experiment, 1984, N2, p.200-203). Other constructions of collimators satisfying the above conditions can also be used. The shape and the size of the penetrating radiation beam formed by the collimator are determined by the character of the investigated object.

The registering device represents a position-sensitive sensor of x-ray radiation allowing for the measurement of the intensity of the scattered radiation. It can be any space-sensitive two-coordinate detector possessing the needed spatial resolution and the sensitivity towards the falling radiation. It is preferable to have a detector with high spatial resolution. the sensitivity of the detector determines the needed power of the radiation source and the velocity of the object scanning.

Between the investigated object and the detector there is a space filter. It is situated in such a way that it can overlap the primary radiation beam and provide the passage of coherently scattered on ultra small angles radiation near the frontiers of the primary beam, on the detector. The areas of the space filter, overlapping the transparent collimator's regions, are made from a material which is opaque for the falling radiation and has its own low background of scattering. The borders of the space filter have the shape providing low level of scattering of the radiation falling on them. In order to determine the level of the intensity of primary beam radiation, passed through the object, a row of detectors is installed on the opaque areas of the space fitter. Those detectors measure the intensity of the falling radiation and do not block the passage of the radiation scattered by the object through transparent areas of the space filter.

The space filter can be situated just beyond the investigated object. It allows to decrease the total level of noise during the registration of the scattered radiation, at the expense of screening the primary beam's radiation scattered in the air and the elements of the device. However it necessitates a high precision of positioning the space filter relatively to the primary beam. The space filter can be situated just in front of the detector, registering the scattered radiation, or occupy any intermediate position between the investigated object and the detector.

All the data received during transillumination of the investigated object from different directions, enter the system of information processing. When processing the data of coherently scattered radiation the optical thickness of the object on the way of the examining beam is taken into the account. The restoration of the object image from the absorbed and the scattered radiation is made with the use of known principles of computerized tomography.

Another variant of the scheme of the above device allows to use more effectively the source radiation. This variant contains a source of penetrating radiation, a collimator forming the flow of radiation falling on the object in the shape of several narrow low-expansion beams, a space filter situated beyond the object and a position-sensitive detector. The collimator is made in the form of a regular periodical structure with alternating slit-like areas transparent for the radiation and opaque areas.

The formed rays overlap a separate stripe in the object's projection. The space filter is regular periodical structure similar to the collimator, in which the areas corresponding to the transparent collimator's areas, are made of a material opaque for penetrating radiation, so that opaque areas of the filter overlap transparent areas of the collimator. With this the size of channels (or slits) and the period of collimator's structure , as well as the size of transparent areas of the space filter must provide the registration of the radiation scattered under ultra small angles, on the position-sensitive detector. The detectors placed on the opaque areas of the space filter allow to determine the intensity of radiation of the primary beams passed through the object. The shape and the position of the channels can be different: for example, slits, round openings situated in a hexagonal package, etc., which is determined by the character of the objects studied in the plant. The following requirements are common for such type of collimators: firstly, the lines of the surfaces forming the transparent channels, must converge on the focus spot of the source for the increase of the effectiveness of the use of ray energy of the plant; with this the radiation in different channels of the collimator can come from different areas of the source's focus spot (the possibility of use of powerful wide-focus sources of radiation); secondly, the collimator must form the beams with such a width and dispersion γ that it would be possible to register the radiation scattered in small-angled diapason, that is, any ray scattered by the object under the small angle α must go out of the frontiers of the primary beam in registration zone; thirdly, the collimator's structure must have such a period, that would make impossible the overlapping of adjacent beams in the plane of the detector; it would allow to register the scattering in small angles, up to the β angle (α and β - the angles determining the registered small angled diapason: α can be of 5 angled seconds and more, β - up to 1 degree).

In order to satisfy those requirements the collimator's input and output have to be pulled apart to a distance that would be significantly greater than the collimator's aperture size. From the constructive viewpoint a slit collimator can be executed in the form of alternating radiation-opaque plates and clearances between them, or in the form of two diaphragms - with one or more slits at the input and multi-slitted at the output - positioned in the due order, etc. Similarly the collimator with radiation-transparent channels with round aperture can be executed constructively in the form of a capillary plait or two diaphragms: the input diaphragm with one or several openings, and the output diaphragm with multiple openings.

The spatial filter is a response regular periodical structure for the collimator, that is, it is constructed in such a way that it detains the direct beams formed by the collimator and lets pass the radiation scattered in the object's plane under small angles in angled diapason from α to β. Constructive execution of the spatial filter must correspond to the collimator used: a linear collimator necessitates a spatial filter in the form of linear raster; a collimator with densely packed cylindrical channels - in the form of a raster with round openings and hexagonal cell.

Another variant of the device foresees the use of a spatial filter, which is semi-transparent for the falling radiation. In such case the spatial filter is made of a material with low level of radiation scattering and able to weaken the intensity of the radiation passed through the object, by the known number of times, to the level of the intensity of the scattered radiation. It is preferable to have an intensity of the radiation at the frontier of the primary beam, weakened by the spatial filter, which would be of one order less than the intensity of the radiation scattered by the object on ultra small angles near the frontier of the primary beam. In such case a position-sensitive detector, situated beyond the spatial filter, registers simultaneously the intensity of the radiation in the beam and of the radiation scattered by the object on ultra small angles.

Another variant of the device for computerized tomography gives the possibility the determine the scattering properties of the investigated object, starting with scattering angles of several seconds. It allows to sense structural elements with big period and to decrease significantly the dose of object's irradiation. The essence of physical method used in the described device for registering the radiation scattered on smallangles, consists in the following: a beam of penetrating radiation point-shaped or hachure-shaped in section, is registered by a high-resolution position-sensitive detector. The distribution of the radiation intensity in the detector's plane will be determined by the optical transmitting function of the device. When the object is put into the device, the total optical transmitting function of the device, and, subsequently, the distribution of the radiation intensity in the detector's plane will be changed. The change of the form of the distribution of radiation intensity will be determined by the scattering function of the object.

One of the variant of the devices, working on this principle, can be represented
by a system consisting of a source of x-ray radiation, one or several collimators, and a high-resolution bi-coordinate detector. Each of the collimators forms a flat fan-shaped beam of radiation. The angled distribution of the intensity in this beam in one direction has the form approaching the δ-function, in another direction it will overlap the whole area under investigation. The high-resolution detector measures the distribution of the radiation intensity in the x-ray beam in the presence and in the absence of the object. In order to receive precise measurements it is necessary to have separate sensitive elements of the detectors whose dimensions would be less than the half-width of the x-ray beam distribution in the registration plane, preferably by an order. The detector must register angled distribution of the intensity in the primary beam up to the angle of several tens of minutes. Such way of measuring allows to register the x-ray scattered under small angles, not only those passing over the beam's frontiers, but also those leading to the re-distribution of intra-beam radiation. In order to have the possibility to compare insignificant changes of the big signals during the data processing, the obtained indices of the distribution of radiation intensity in the beam in the presence and in the absence of the object are normalized for the total intensity of the radiation falling on the object and passing through it, respectively. Thus, the obtained data are reduced to the common conditions, and the changes in the form of the curve of distribution of the radiation intensity in the beam (the difference of the normalized spatial distribution of the intensity) will reflect the scattering function of the medium, through which the radiation is passing; with this simultaneously the coefficient of medium absorption will be determined.

The optimal conditions of registration during the investigation of different objects can be achieved by selecting the stiffness, that is the length of the wave of the used penetrating radiation. The softer is the radiation (the bigger is the wave's length), the greater will be the changes of the normalized curve of the intensity distribution in the penetrating beam beyond the object; however with this the part of radiation absorbed by the object increases, while the signal on the detector decreases. The choice of optimal parameters of the penetrating radiation depends on the character of the investigated object, and is effectuated individually in each case. With the use of polychromatic source of radiation it can be achieved either by selecting a filter sectioning the needed spectral diapason of the radiation, or by using a detector selectively sensitive to a particular diapason of the energy of the registered quanta. In the latter case each spectral diapason of the penetrating radiation has its own intensity distribution in the post-object beam registered on the detector. The common requirement for the detectors used in such scheme of simultaneous registration of the radiation scattered and passed through the object consists in their ability to measure the radiation intensity in wide dynamical diapason of values. For example, the intensity of scattered radiation is smaller than the intensity in the passed beam by 10³ - 10⁵ times. The detector must measure all this diapason of values of the radiation's intensity.

Another variant of the scheme allows to determine the scattering and the absorbing properties of the investigated object using a wide beam of penetrating radiation. This variant of the scheme allows to use effectively the radiation from the source. Its peculiar feature is the collimator being a multi-slit periodical structure, which forms the flow of x-ray radiation as a wide beam, modulated with high spatial frequency. The detector possessing high spatial resolution in the registration plane measures periodically modulated distribution of the radiation intensity in the presence and in the absence of the object. The presence of the object in the device leads to the change of the function of modulating the intensity distribution in the detector plane. It allows the determine the following parameters of the investigated substance: the decrease of the average value of intensity along the direction of the beam's modulation determines the magnitude of the radiation absorption be different part of the object, while the change of the depth of the modulation of intensity distribution shows indirectly the function of the object scattering. In order to reveal several heterogeneity, occupied be the investigated substance, within the object, it is necessary to have the period of spatial modulation of the radiation in the object smaller than the size of this heterogeneity.

The sensitivity of the described device to the registration of the intensity of the scattered radiation is determined by the spatial frequency and the depth of the modulation of the falling radiation and the resolution of the detector used. The higher is spatial frequency of the radiation's modulation and the bigger is the depth of the modulation, the greater will be the changes of the function of the radiation intensity distribution with the introduction of the object. However maximal values of acceptable spatial frequency of the radiation's modulation are limited by the parameters of the modulator used and the resolution of the registering elements. The spatial sensitivity of the detector must be smaller than the period of the spatial modulation of the radiation, preferably by an order. It is also necessary to have a detector sensitive to the registration of the radiation in wide dynamical diapason of the intensity's values.

In all the devices descried above one can use the beam of penetrating radiation of different forms, depending on the character of the object under study. For example in order to investigate the objects having an anisotropy of the scattering properties (anisotropic structure) it is necessary to have a device able to register the object scattering in at least two mutually perpendicular directions. For this purpose one can use: a point-shaped beam of penetrating radiation, two mutually perpendicular beams flat fan-shaped beams with the same direction of diffusion, etc.

The essence of the invention can be explained by the following figures of the drawings, where:
fig. 1 shows one of the variants of a tomographic device, in which the beam of penetrating radiation going in the direction perpendicular to the plane of the optical system's rotation, overlaps totally the investigated area of the object;
fig.2 shows a device in which the displacement of the system "source -collimator - spatial filter - detector" relatively to the object is performed in spiral trajectory;
fig.3 shows the scheme of a tomograph, in which several identical systems "source - collimator - spatial filter - detector" are used;
fig.4 shows a tomographic device in which the system "source - collimator - detector" is displaced in the trajectory, laid on the surface of a sphere situated around the investigated area of the object;
fig.5 shows one of the dark-field schemes for simultaneous registration of small-angled scattered radiation and the primary beam radiation according to the given image;
fig.6 shows the same scheme of simultaneous registration of small-angled scattered radiation and the primary beam radiation with a semi-transparent trap put on the way of the primary beam; this trap decreases the level of intensity in this beam to the level of intensity of the scattered radiation;
fig.7 shows the scheme with multi-slit collimator and spatial semi-transparent filter in front of the detector;
fig.8 shows another scheme of simultaneous registration of small-angled scattered radiation and the primary beam radiation, in which the detector measures the distribution of the radiation's intensity in the primary beam beyond the object;
fig.9 shows another scheme of registration with spatial modulation of the radiation falling on the object.

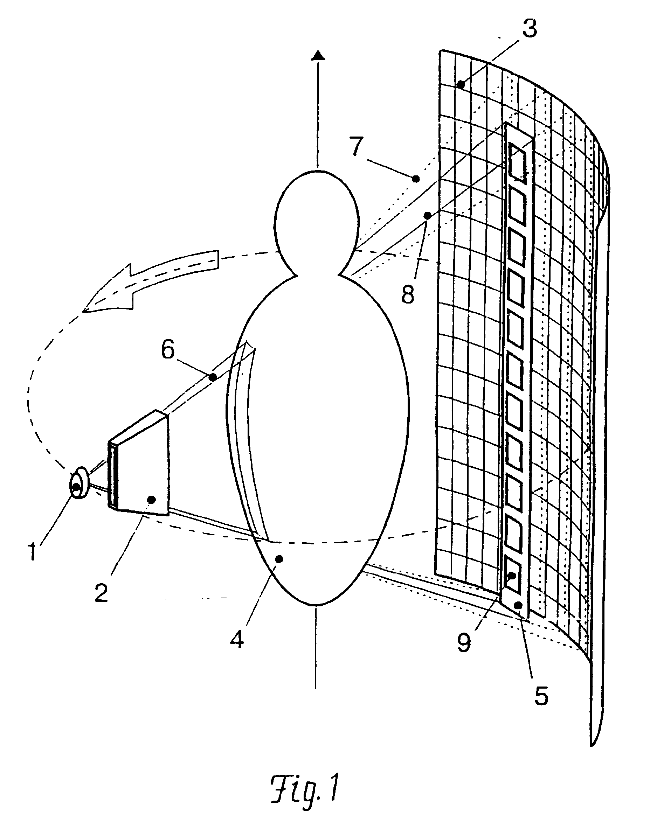

One of the variants of the device used for the imaging of the electron density distribution in the investigated object is a small-angled tomograph shown in fig. 1. It includes a source of radiation 1, a collimator 2, a spatially-sensitive detector 3 and a spatial filter 5 situated between the investigated object 4 and the detector 3. A fan-shaped beam 6 of penetrating radiation, formed by the collimator 2, has in one direction (the plane of rotation) a width and an angled divergence, which provide the registration of small-angle scattering, starting from the angle α (α- cam be of several angled seconds). In perpendicular direction the beam overlaps the whole area under investigation. The scattered radiation 7 is registered by the detector 3 in the direction perpendicular to the beam's plane. The radiation 8 of the primary beam beyond the object is registered by the ruler of the detectors 9, situated on the spatial filter 5. The resolution of spatial heterogeneity in the direction, perpendicular to the fan-shaped beam's plane, are determined by the beam's width; while along the beam they are determined by the magnitude of sensitivity of the detector's sensitive elements. The system of displacement (not shown on the drawing) provides the 360° rotation of the source, the collimator, the spatial filter and the detector around the investigated object 4. During one cycle of measurements the system makes one or several turns, with this under each angle of the object transillumination, the passed radiation 8 and the scattered radiation 7 are registered. A computerized system processes the obtained data and normalizes the scattering (the distribution of the electron density) and the absorbing properties for each area of the investigated object. As a result the reconstruction of the object's internal structure is done.

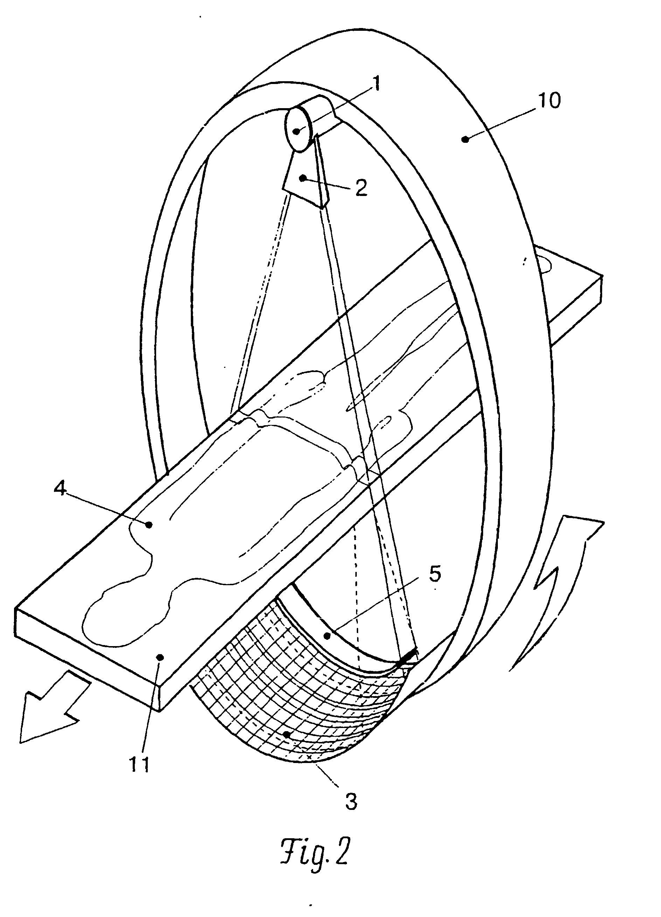

Another variant of the device, shown on fig.2, stipulates the creation of three-dimensional image of the internal structure of the investigated object 4, possessing significant dimensions in one direction. With this the optical system - a source 1, a collimator 2, a spatial filter 5, a detector 3 - is displaced in spiral relatively to the investigated object 4. For example, it can by a system: source 1, collimator 2, spatial filter 5, detector 3, similar to that described above. The spatial filter is made of a material which is semi-transparent for penetrating radiation, and it decreases the radiation intensity in the primary beam beyond the object to the level of the intensity of the radiation scattered under small angles. The collimator 2 is situated in such a way, that the plane of the fan-shaped beam, formed by it, lays in the plane of rotation. Transversal dimensions of the x-ray beam must be bigger than those of any area of the investigated object. The optical system is situated on a rigid frame 10, which can make a 360° turn around the investigated object. During the rotation of the frame 10 a transporting bed 11, with the investigated object 4 on it, is displaced along the axis of rotation. The beam of penetrating radiation passes sequentially through each area of the object from all the directions (360°). The velocity of the object displacement is determined by the rate of rotation of the optical system and the sensitivity of the detector 3. The isolation and the processing of the signal corresponding to the small angle scattering and the radiation passed through the object is made similarly to the device described above.

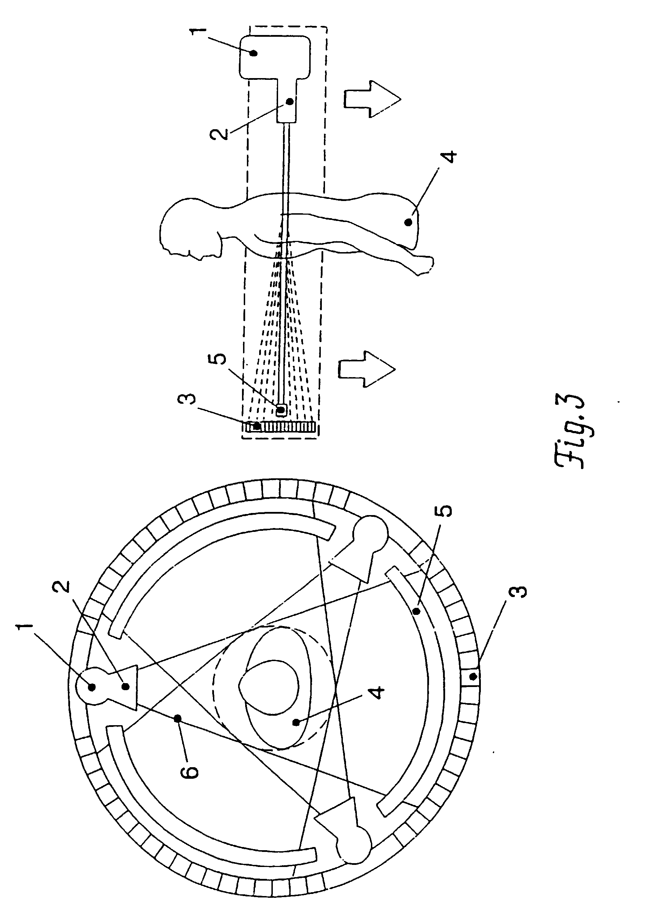

The device presented on fig.3, stipulates the presence of several identical schemes "source 1, collimator 2, spatial filter 5, detector 3" situated at different angles relatively to the object 4. For example, it can be a device including three or more identical systems, analogous to those described above, situated evenly under different angles in the same plane. The fan-shaped beams 6, formed by each of the systems, lay in one plane, corresponding to the plane of the systems themselves, and overlap the whole object's area under investigation. The reconstruction of the internal structure of the transilluminated object's area is made by comparison of the data obtained from each system. In order to receive a three-dimensional image of the object's internal structure, the investigated object and the device are displaced one relative to another. For example, the device can be displaced as a whole (the plane of the systems) along the long axis of the object 4. The isolation of the data, corresponding to the radiation scattered and absorbed by the investigated object, as well as the reconstruction of three-dimensional image of the object are made similarly to the devices described above, the use of several systems allows to increase the velocity of data acquisition.

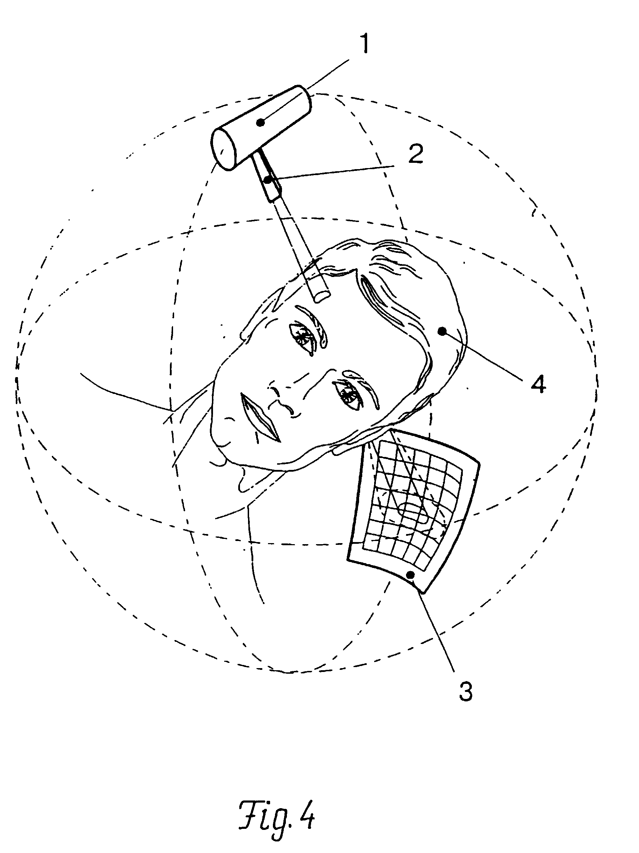

Fig 4 shows a device for computerized tomography, based on the principles of the reconstruction of the object's internal structure from the data of scattered and absorbed radiation. It consists of a source of radiation 1, a collimator 2, a high-resolution position-sensitive detector 3. The collimator forms a point-shaped or a hachure-shaped beam for transilluminating the investigated area of the object. With this the optical system "source of radiation 1, collimator 2 and defector 3" is displaced in a complex trajectory, laid on the surface of a sphere situated around the investigated area of the object 4. The beam, formed by the collimator, must satisfy the conditions, necessary for the registration of the radiation scattered under small angles, and simultaneously, the registration of the radiation passed through the object. The isolation of the data corresponding to the radiation scattered and absorbed in the investigated object, is realized according to one of the schemes described above. For example, as for the determination of the changes of the radiation intensity in the primary beam beyond the object. With this the sensitive elements of the detector 3 must be smaller than the semi-width of distribution of the x-ray beam intensity in the registration plane, preferably by an order. The number of projections of the investigated area of the object, obtained during the displacement of the optical system in its trajectory, must be sufficient to form a three-dimensional image of electron density distribution in this area. After the data processing the computerized system forms a three-dimensional image of the investigated are of the object. Such device can be used, for example, for brain tomography.

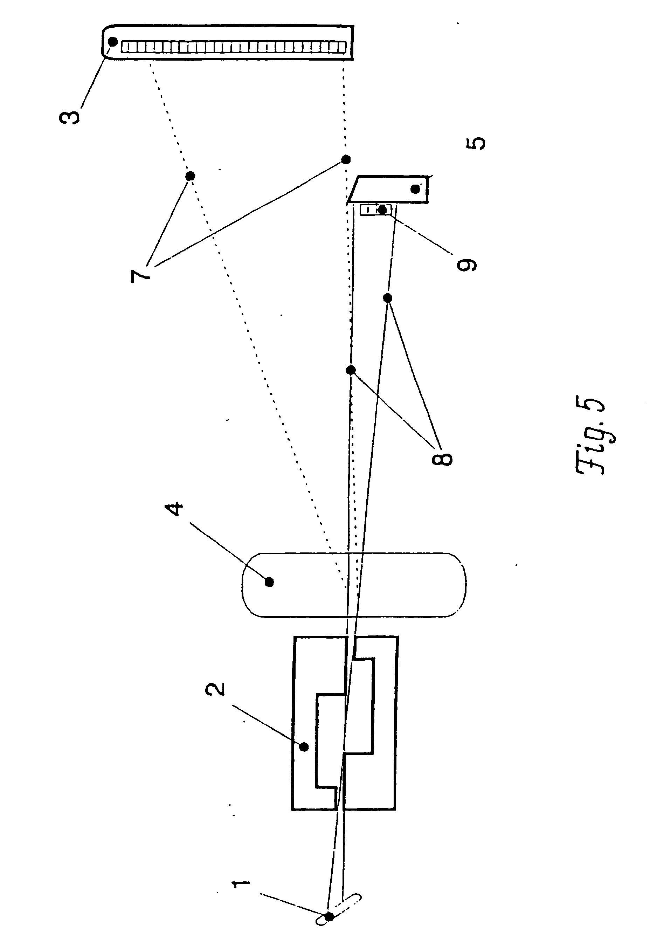

On figs. 5-9 one can see different variants of optical schemes for simultaneous registration of the radiation scattered under small angles, and the primary beam radiation beyond the object.

One of the variants of such scheme (fig.5) can be composed of a source of x-ray radiation 1, a collimator 2 made, for example, according to Kratky scheme and forming the radiation as a flat fan-shaped beam with such a width and an angled divergence in at least one direction, that it can register the scattered radiation ins mall-angled diapason, while in another direction the formed beam must overlap the whole investigated area of the object 4, a spatial filter 5 and a bi-coordinate position-sensitive detector 3. The detectors 9, measuring the radiation intensity in the primary beam beyond the object, are situated on the spatial filter 5. With this the detectors 9 must be of such dimensions and must be situated in such a way, that does not influence the registration of the radiation 7 scattered by the object 4, by the bi-coordinate detector 3.

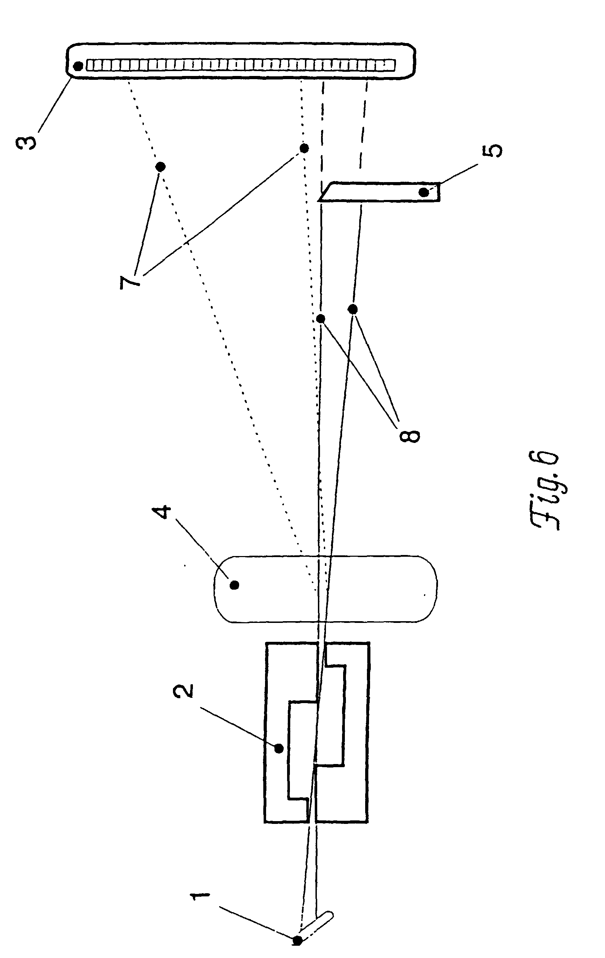

Fig.6 shows another variant of the scheme for simultaneous registration of the radiation scattered 7 and passed 8 through the object 4. This scheme stipulates the introduction of the radiation 8 of the beam from the spatial filter 5 passed through the object, into the channel, so that the intensity of radiation on the frontiers of the primary beam beyond the spatial filter 5 would be of one order smaller than the intensity of the scattered radiation 7 near the frontiers of the primary beam. With this the function of the radiation scattering by the object is determined directly by the data obtained from the detector 3, while the intensity of the radiation passed through the object, is calculated with a known coefficient of the radiation absorption be the filter.

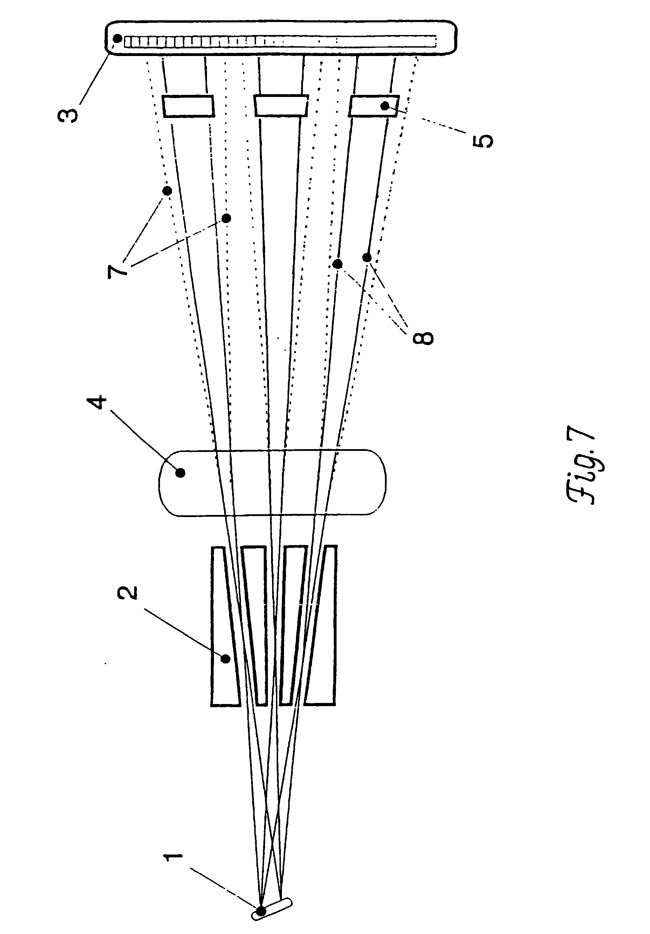

Another variant of the scheme (fig.7) for simultaneous registration of radiation scattered 7 and passed 8 through the object 4, can contain a source of penetrating radiation 1, a collimator 2, forming the flow of radiation falling on the object in the form of several narrow low-expanding beams, a spatial filter 5 situated beyond the object and a position-sensitive detector 3. The collimator 2 is made in the form of a regular periodical structure with radiation-transparent slit-like or channel-like areas alternating with opaque areas.

The formed rays overlap a separate stripe in the projection of the object 4. The spatial filter 5 is regular periodical structure, similar to this one of the collimator 2, in which the areas corresponding to the transparent areas of the collimator, are made of a material, semi-transparent for the penetrating radiation in such a way, that the semi-transparent areas of the filter 10 overlap the transparent areas of the collimator 2. With this the dimensions of the channels (or slits) and the period of the structure of the collimator 2, as well as the dimensions of transparent areas of the spatial filter 5 must provide the registration of small-angles scattered radiation 7 and, separately, of the weakened radiation passed 8 through the object 4, by the position-sensitive detector 3. The form and the position of the channels are determined by the character of the investigated objects. The following requirements are common for such type of collimators: firstly, the lines of the surfaces, forming transparent channels, must converge on the source's focus spot in order to increase the energetic efficacy of the plant; with the radiation into different channels of the collimator 2 can come from different areas of the focus spot of the source 1 (the use of powerful wide-focus radiation sources); secondly, the collimator must form the beams with such a width and a divergence γ that it would be possible to register the radiation scattered in small-angled diapason, that is, and ray scattered by the object under a small angle α would pass out the frontiers of the primary beam within the registration zone; thirdly, the period of the collimator's structure must be such, that it would make impossible for adjacent beams to overlap each other in the detector's plane; it allows to register the scattering in small angles, up to the β angle (α and β - the angles determining the registered small-angle diapason; α can be of 5 angled seconds and more, β - up to 1 degree).

From the constructive viewpoint the collimator can be executed, for example, in the form of alternating radiation-opaque plates and clearances between them.

A spatial filter of small-angles radiation is a response regular periodical system for the collimator, that is, it is constructed in such a way, that it weakens the direct rays, formed by the collimator, and lets through without any hindrance the radiation scattered in the object's plane under small angles within the angled diapason from α to β. The constructive execution of the spatial filter must correspond to the collimator used: the spatial filter for a linear collimator must be executed in the form of a linear raster. The magnitude of the weakening of the primary beam radiation is determined by the spatial filter's coefficient of absorption.

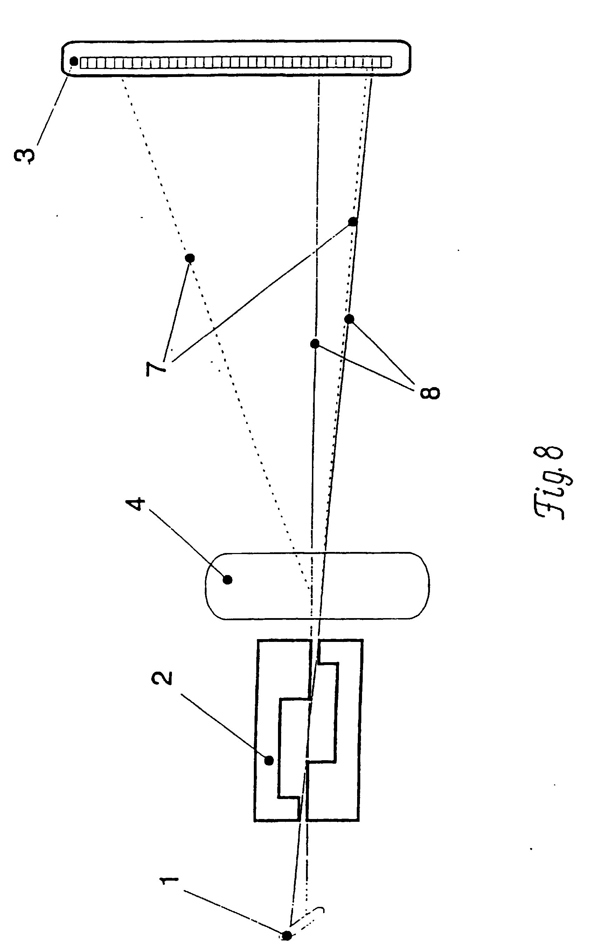

Fig. 8 shows another scheme for simultaneous registration of the small-angled scattered radiation and the primary beam radiation; in this scheme a high resolution detector 3 measures the distribution of the intensity of radiation in the x-ray beam in the presence 7 and in the absence 8 of the object 4. In this case the collimator 2 forms the radiation of the source 1 as a flat fan-shaped beam, which has in one direction the angled distribution of intensity close by its form to the δ-function, and in the other direction it overlaps the whole investigated area of the object. In order to provide precise measurements it is necessary to have the dimensions of separate sensitive elements of the detector smaller than the semi-width of the distribution of the x-rat beam 8 intensity, preferably by one order. Such way of measuring allows to register the x-ray 7 scattered under small angles, not only going out of the beam's frontiers, but also those, which lead to re-distribution of the radiation intensity inside the beam. In order to have the possibility to compare insignificant changes of big signals during the data processing, the obtained distributions of the radiation intensity in the beam in the presence and in the absence of the object are normalized for the total intensity of the radiation, falling on the object and passed through it, respectively. Thus, the obtained data are led to common conditions, and the change of the form of the curve of the radiation intensity distribution in the beam (the difference of normalized spatial distribution of intensity) will reflect the scattering function of the medium through which the radiation passes. The common requirement to the detectors with this scheme of simultaneous registration of the radiation, scattered 7 and passed through the object 8, is their capacity to measure the intensity of radiation in the wide dynamical diapason of values.

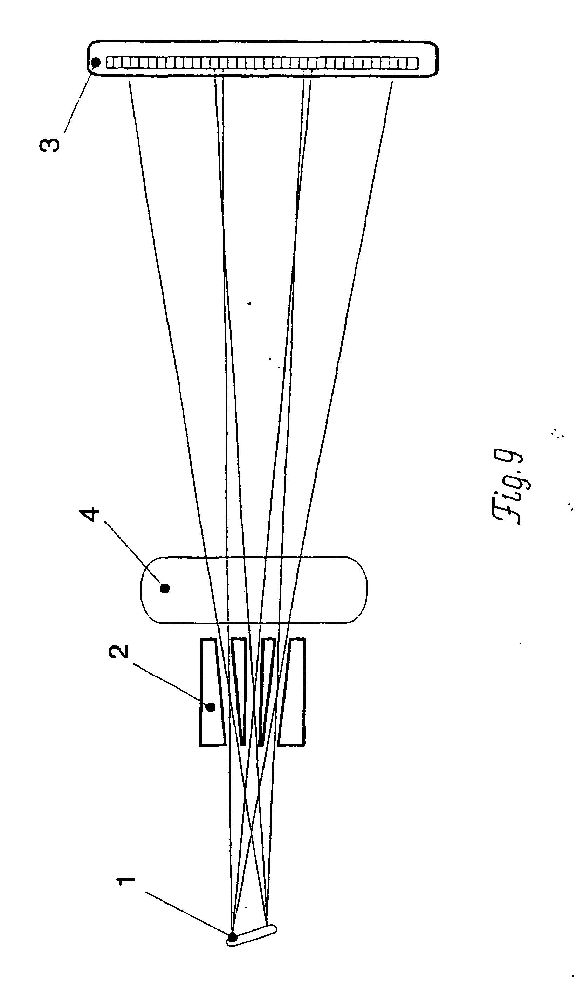

Another variant of the scheme (fig.9) allows to determine the scattering and the absorbing properties of the investigated object using a wide beam of penetrating radiation. This variant allows to use more effectively the radiation from the source 1. It differs in that the collimator 2 represents a multi-slit periodical structure, forming a flow of x-ray radiation as a wide beam, modulated with high spatial frequency. The detector 3 with high spatial resolution in the registration plane, measures the periodically modulated distribution of the radiation intensity in the presence and in the absence of the object 4. The presence of the object 4 in the device leads to the change of the function of modulation of intensity distribution in the plane of the detector 3; it allows to determine the following parameters of the investigated object: the magnitude of absorption of the x-ray radiation by different parts of the object by the decrease of the average value of intensity along the direction of the beam modulation, the scattering function of the object by the change of the depth of modulation of the intensity distribution. In order to reveal the heterogeneity occupied by the investigated substance, within the object, it is necessary to have period of spatial modulation of radiation in the object smaller than the size of heterogeneity itself.

The sensitivity of the described device for the registration of the intensity of the scattered radiation is determined by the spatial frequency and the depth of modulation of the falling radiation, as well as by the resolution of the detector used. The higher is the spatial frequency of the radiation's modulation and the bigger is the depth of modulation, the greater are the changes of the function of the radiation intensity distribution with the introduction of the object. However maximal values of acceptable spatial frequency of the radiation modulation are limited by the parameters of the modulator used and the resolution of the registering elements. The spatial sensitivity of the detector must be smaller than the period of spatial modulation of the radiation, preferably by one order. It is also necessary to have a detector sensitive for the registration of the radiation within wide dynamical diapason of the intensity values.

## Claims

1. A device for small-angle computerized tomography, containing a source of penetrating radiation, a collimator, forming the radiation flow falling on the object in the form of one or several narrow, low-expanding at least in one direction, beams, a coordinate-sensitive detector performing the registration of coherent radiation scattered on small angles, a system for relative displacement of the complex "source - collimator - detector" and the object, and a computerized system for processing the information obtained from the coordinate-sensitive detector, differing by a spatial filter put between the object and the coordinate-sensitive detector and separating the radiation scattered by the object on ultra-small angles relatively to the direction of the falling beam.

2. A device in p.1, differing by a collimator executed in the form of a regular periodical structure with radiation-transparent slit-like or channel-like areas alternating with opaque areas and overlapping a separate stripe in the object projection; the spatial filter represents a collimator-like regular periodical structure, in which the areas corresponding to the transparent areas of the collimator, are made from a radiation-opaque material, and the areas overlapping opaque areas of the collimator, are made transparent for penetrating radiation; on opaque areas of the filter there are detective elements for the measurement of radiation passed through the object; with this the sizes of the channels or the slits and the periodical structures of the collimators must provide the registration of the radiation scattered on ultra-small angles, by the position-sensitive detector.

3. A device in p.1, differing by a collimator executed in the form of a regular periodical structure with radiation-transparent slit-like or channel-like areas alternating with opaque areas and overlapping a separate stripe in the object projection; the spatial filter is situated in front of the detector and represents a collimator-like regular periodical structure, in which the areas overlapping opaque areas of the collimator, are made transparent for penetrating radiation, and the areas overlapping transparent areas of the collimator are made of the material, which partially absorbs the radiation and decreases the intensity of the radiation passed through those areas to the level of the radiation scattered on small angles and passed on the coordinate-sensitive detector through transparent areas of the spatial filter.

4. A device for small angle computerized tomography, containing a source of penetrating radiation, a collimator forming the radiation flow falling on the object in the form of one or several narrow, low-expanding, at least in one direction beams, a detecting system, a system a system for relative displacement of the complex "source - collimator - detector" and the object, and a computerized system for processing the information obtained from the coordinate-sensitive detector, installed at such a distance from the object and having such spatial sensitivity, which allows to register angled distribution of the intensity on the section of the beam passed through the object with spatial resolution which is more narrow, than the semi-width of the intensity distribution in the beam in the registration plane; with this each beam in formed by the collimator in the object projection is, at least in one direction, more narrow than the area occupied by the controlled substance within the object.

5. A device for small angle computerized tomography containing a source of penetrating radiation, a collimator forming the radiation flow falling on the object in the form of one or several narrow, low-expanding, at least in one direction beams, a detecting system, a system a system for relative displacement of the complex "source - collimator - detector" and the object, and a computerized system for processing the information obtained from the coordinate-sensitive detector, differing by a collimator representing a slit-like structure , forming a set of narrow, low-expanding beams of radiation in the direction of the investigated object; the registration of the radiation passed through the object is made by the bi-coordinate space-sensitive detector and a block for information processing connected with the detector; with this the period of multi-slit structure is chosen based on the condition of providing a period of spatial modulation of the radiation which is at least by two times smaller than the size of the area, occupied by the controlled substance within the object, and the spatial resolution of the detector is smaller than the period of spatial modulation of the radiation in the registration plane.

6. The device in one of the pp. 1-5, differing by each beam overlapping the whole investigated area of the object in one direction, with this the complex "source - collimator - detector" is executed with the possibility of performing a 360° rotation relatively to the investigated object in the plane perpendicular to the plane of the fan-shaped beam.

7. A device in one of the pp. 1-5, differing by the complex "source - collimator - beam" executed with the possibility of spiral displacement relatively to the investigated object.

8. A device in one of the pp.1-5, differing by the collimator forming a beam with point-shaped or a hachure-shaped section, with this the complex "source - collimator - detector" is executed with the possibility of displacement in complex trajectory laying on the surface of a sphere situated around the investigated area of the object.
